# EUROPEAN PATENT APPLICATION

(11) **EP 4 379 063 A1**
(43) Date of publication of application: **05.06.2024**
(21) Application number: 22306756.2
(22) Date of filing: 29.11.2022
(51) Int. Cl.: C12Q 1/6806, G01N 35/00, B82Y 10/00

(54) **METHOD OF MARKING A MATERIAL FOR AUTHENTIFICATION AND/OR TRACEABILITY PURPOSES**

(71) Applicant: Institut national de recherche pour l'agriculture, l'alimentation et l'environnement, 75007 Paris (FR); Centre National de la Recherche Scientifique, 75016 Paris (FR); Institut National des Sciences Appliquées de Toulouse, 31400 Toulouse (FR)
(72) Inventor: The designation of the inventor has not yet been filed
(74) Representative: Jacobacci Coralis Harle

(57) **Abstract**

The invention relates to a method for marking a material, for example for purposes of authentication and/or traceability of said material, notably batches generated.

The said marking method comprises:
- a step of supplying a marking composition chosen among molecular tags, and
- a step of incorporating said marking composition into said material.

And, according to the invention, said marking composition comprises particles, advantageously remaining intact after incorporation in said material, which comprise:
- a homogeneous porous matrix which comprises a plurality of pores,
- an information support added to said homogeneous porous matrix, wherein said information support consists of a polymer comprising an information sequence, and
- a particle surface having a coating with at least one fluidizing agent, suitable for promoting uniform dispersion of said particles within said material.

## Description

### Technical field of the invention

The present invention relates to the technical field of marking a material with molecular tag(s), for example for purposes of authentication and/or traceability of said material.

### Prior art

Marking methods are positioned in the context of two main applications, conferring competitive advantages on materials or finished products, i.e.:
- authentication to protect notably against counterfeiting, which is the cause of commercial losses, and
- traceability, as brands and retailers are increasingly under pressure from consumers to create transparency covering their entire supply and production chain.

A typical marking method is based on conventional barcodes, QR codes (Quick Response Code) or even RFID (Radio Frequency Identification) chips, which are authentication and batch tracking systems positioned on the packaging or in surface of the products and which have rapid reading systems.

However, these "surface" tags cannot be applied to some particular materials or in scenarios where the code should be invisible to the naked eye.

Molecular tags address these shortcomings via their nanoscale footprint and difficulty to forge.

Indeed, "molecular tags" are constituent element of the molecular composition of the materials in which they are incorporated. They are an integral part of the mass of the material of interest.

Molecular tags are thus a real molecular marker making it possible to trace back to a batch of material and to follow its evolution over time.

This authentication technology provides also added value to materials. It collects data from tagged materials and tracks them as they are incorporated into finished products.

Thus molecular tags offer an innovative solution to physically mark, trace, and authenticate materials from the producer to the retailers of products made with these materials, thus creating transparency throughout the supply chain (linear or circular).

However, the current molecular tags are not totally satisfactory.

For example, WO2013143014 discloses encapsulation of informative DNA in non-porous silica nanoparticles suitable for an invisible secure marking or information which is a part of a product.

But silica nanoparticles have non-negligible solubility in water at basic pH values (above pH 9), so silica shells can be instable in alkaline materials and the dispersion of colloidal silica nanoparticles is affected.

Therefore, there is a need of molecular tags which can be well dispersed, preferably as a uniform dispersion, within the mass of the targeted materials, without altering their properties.

Moreover, preferably, the information support needs to be protected in the material and then to be recovered with the information sequence associated.

The process for extracting information from products made, elaborated with the target material, must be simple enough to be transposed into analysis laboratories.

### Presentation of the invention

In order to remedy the aforementioned drawback of the state of the art, the present invention proposes a method for marking a material, for example for purposes of authentication and/or traceability of said material, notably batches generated. The marking method comprises:
- a step of supplying a marking composition chosen among molecular tags, and
- a step of incorporating said marking composition into said material.

And, in accordance with the invention, said marking composition comprises particles, advantageously remaining intact after incorporation in said material, which comprise:
- a homogeneous porous matrix which comprises a plurality of pores,
- an information support added to said homogeneous porous matrix, and
- a particle surface having a coating with at least one fluidizing agent, suitable for promoting uniform dispersion of said particles within said material.

Such a marking method is particularly interesting since the particles of the marking composition are particularly well dispersed within the mass of the target material, without altering their properties. This optimized dispersion is particularly interesting to increase the sensibility when recovering the molecular tags in the material.

Moreover, the information support is advantageously well protected in the material, and then recovered with the information sequence associated.

Preferably, the information support is stabilized by the association with the porous matrix and the fluidizing agent, which leads to protection within the material, and then recovered with the information sequence associated.

The use of a homogeneous porous matrix has a definite advantage because it is possible to load a lot more information support for particles of comparable composition and size.

Addition of information support to porous material, and surface modifications with fluidizing agent, assure the stability of encoded data and its applicability to a range of materials.

Finally, preferably in another advantageous aspect, the particles are stable in the material, but they can be degradable if exposed to the environment. In addition, decomposition products advantageously result in molecules of environmental concern and safety with regard to human health.

Other non-limiting and advantageous characteristics of the process in accordance with the invention, taken individually or in all technically possible combinations, are the following:
- the homogeneous porous matrix is composed of at least 90% w/w of salts, preferentially inorganic salts, consisting of an ionic assembly of positively charged cations and negatively charged anions or consisting of a metal oxide, wherein said homogeneous porous matrix is insoluble in the said material;
- the particles are decomposable by metal chelating agent or by specific pH conditions (as acidic or alkaline solutions);
- the particles have a size ranging from 200 nm to 100 µm, preferably from 500 nm to 10 µm, more preferably from 750 nm to 3 µm;
- the particles have pore size ranging from 2 nm to 500 nm, preferably from 2 to 200 nm, more preferably ranging from 25 nm to 150 nm;
- the information support is included within the pores and/or absorbed on the surface of the homogeneous porous matrix;
- the information sequence of said information support results from a transcoding of a system of ordered bits, the latter resulting from an encoding of a set of raw data;
- the information support is chosen among nucleic acids and nucleic acids analogs, preferably DNA and more preferably single stranded DNA or double stranded DNA; the information support consists advantageously of nucleic acids fragments, preferably having a size of less than 500 bp, preferably ranging from 50 to 250 bp;
- the information support is associated, preferably adsorbed and/or included, with the said homogeneous porous matrix;
- the fluidizing agent is chosen among surface-treating agents of inorganic fillers (see KATZ, Harry S. et MILESKI, J. V. (ed.). Handbook of fillers for plastics. Springer Science & Business Media, 1987; for inorganic fillers overview such as precipitated calcium carbonate, barite, kaolin, talc, silica, and carbon black), which includes saturated fatty acids, advantageously having a carbon chain length of from C15 to C20, e.g. palmitic acid and stearic acid; unsaturated fatty acids, advantageously having a carbon chain length of from C15 to C20, e.g. oleic acid and linoleic acid; phospholipids, e.g. phosphatidylcholine, phosphatidylethanolamine, phosphatidylserine; lipids mixtures, preferably biosourced or biodegradable, e.g. lecithin, beeswax; resin acids, e.g. alicyclic carboxylic acids and abietic acid as well as salts, esters and ethers thereof; polymers, e.g. polycarboxylic acids, fused phosphoric acid, polyacrylate, polyamine, polyacrylamide, poly-aminoacyls, polystyrene, polysaccharide (dextran, alginate, chitosan, chondroitin, cellulose, pectin etc.), lignin, preferably biosourced or biodegradable polymers; others inorganic salts, titanates, silanes, siloxanes, alkenyl succinic anhydrides surface treating agents;
- the material is chosen among liquid, semi-solid or solid materials, for example plastic, thermoset, varnish, rubber, paint, oil, pharmaceuticals;
- the step of incorporating consists of incorporating the marking composition into the material during the fabrication process of said material.

The present invention also relates to the marking composition, for marking a material, for example for purposes of authentication and/or traceability of said material, notably batches generated, wherein said marking composition comprises particles, advantageously remaining intact after incorporation in said material, which comprise:
- a homogeneous porous matrix which comprises a plurality of pores,
- an information support added to said homogeneous porous matrix, wherein said information support consists of a polymer comprising an information sequence, and
- a particle surface having a coating with at least one fluidizing agent, suitable for promoting uniform dispersion of said particles within said material.

The present invention also relates to the material comprising the marking composition of the invention.

The different characteristics, variants and embodiments of the invention can be associated with each other in various combinations insofar as they are not incompatible or exclusive of each other.

### Detailed description of the invention

In addition, various other characteristics of the invention emerge from the following description.

The present invention relates to a method for marking a material, for example for purposes of authentication and/or traceability of said material, notably batches generated, with a marking composition.

As used herein, the "material", or "object", relates to a material to be marked (or then marked) with the marking composition comprising at least a molecular tag.

Said material can be the elements, constituents, parts or substances of which something is composed or can be made.

The marking composition is in particular useful in a verification method that allows distinguishing and/or identifying the material.

Thus, verification methods are suitable for distinguishing genuine material from false material.

In addition, verification methods are appropriate for identification of materials (e.g. by batch type, manufacturing site, manufacturing time).

In general, the marking method of the invention aims at conferring competitive advantages on the marked material, i.e.:
- authentication, to indicate the origin of the material and to protect notably against counterfeiting, and
- traceability, enabling manufacturers and trade partners as well as consumers and authorities to identify, authenticate and track goods throughout the entire supply chain.

According to the invention, the marking method comprises the following steps:
- a step of supplying a marking composition chosen among molecular tags, and
- a step of incorporating said marking composition into said material.

According to the invention, starting from a material containing the marking composition, a step of recovering can be implemented on said material to recover the information support.

Preferably, said step of recovering is implemented on the material elaborated products, to extract said information support.

As used herein, "molecular tags" relates advantageously to a compound suitable for identifying and/or authentication of a material of interest.

Molecular tags are constituent element of the molecular composition of the materials in which they are incorporated. They are an integral part of the mass of the material of interest.

In other words, the present invention also relates to a used of said marking composition for marking a material, for example for purposes of authentication and/or traceability of said material, notably batches generated.

### Generality about the marking composition

In the invention, the marking composition comprises particles, consisting of molecular tags, comprising a combination of at least three components:
- a homogeneous porous matrix which comprises a plurality of pores,
- an information support added to said homogeneous porous matrix, wherein said information support consists of a polymer comprising an information sequence, and
- a particle surface having a coating with at least one fluidizing agent, suitable for promoting uniform dispersion of said particles within said material.

Preferably, the marking composition comprises a "matrix-fluidizing agent" couple that is adapted to the information support and to the target material.

As used herein, the term "particles" or "particulates" means the smallest entity that can be identified as a particulate from its appearance among the marking composition.

The particles have advantageously a size ranging from 200 nm to 100 µm, preferably from 500 nm to 10 µm, more preferably from 750 nm to 3 µm.

The term "size" advantageously means a physical characteristic size of the particles linked to a method of measurement (or dimensional analysis by an appropriate technique), for example by laser diffraction or sieving or image analysis.

The size of a solid particle advantageously corresponds to its "equivalent sphere diameter" or "equivalent sphere diameter", that is to say advantageously the diameter in volume (dᵥ) defined as the diameter of a perfect sphere having the same volume in the analysis as the solid particle studied.

The particle size of these particles, and in particular the particle size distribution, is advantageously defined by a value of D₅₀ or dso, also called "median size" or "median diameter".

The D₅₀ represents the particle size for which 50% of the volume (or mass) has a lower (or higher) particle size; in other words, the D₅₀ is the diameter corresponding to 50% of the cumulative frequency in number, mass or volume.

Thus, as used herein, the "median size" with respect to the particles means advantageously the 50th percentile particle diameter (volume median particle diameter, D₅₀) in its size distribution by volume (e.g., measured by a particle size analyzer based on laser diffraction/scattering spectroscopy).

The particle size distributions can be measured by laser diffraction using a Mastersizer 2000 or 3000 equipped with a Hydro2000S system.

In general, the particles form advantageously a protective agent for the information support.

As used herein, the term "protective agent" means molecules that can prevent information support damages caused by environmental factors such as UV light, ionizing radiation, and genotoxic chemicals or caused by naturally occurring agents such as reactive oxygen species, reactive nitrogen species, reactive carbonyl species, lipid peroxidation products and alkylating agents.

The particles of said marking composition advantageously remain intact after incorporation in said material of interest.

As used herein, the terms "remain intact" means that the particles are included in the material of interest, with the information support still attached / associated to the homogeneous porous matrix.

These terms "remain intact" also enclose at least a part of the particles whereof the homogeneous porous matrix is fractionated or divided due to the marking method but wherein the information support is still attached / associated to the homogeneous porous matrix.

In general, the concentration of information support in the particles is advantageously from 0.1 to 500 mg of information support per g of homogeneous porous matrix.

### Homogeneous porous matrix

The homogeneous porous matrix advantageously forms a "vehicle", i.e., a compound that can be loaded with the information support and permits its incorporation in the targeted material.

The homogeneous porous matrix advantageously aims at carrying the information support, by confining the latter, to avoid degradation of said information support by environmental factors or by the material.

As used herein, "homogeneous" or "homogenous" means in particular that said matrix is devoid of any successive layers (e.g., devoid of core-shell).

As used herein, "porous" means that the matrix is a solid with pores, i.e., cavities, channels or interstices, which are deeper than they are wide. The term "porous" also means that the matrix can adsorb information support described herein.

In general, the term "porous", as used herein refers, according also to IUPAC recommendations (Rouquérol, 1994), refers to:
- pores with free diameters in the range of 2 to 50 nm, for mesoporous particles, or
- pores with free diameter having widths larger than 50 nm, for macroporous particles.

The "free diameters" or "pore sizes" of pores refer to the pore diameter measured in the dried state with a measurement technique such as the determination of nitrogen sorption isotherms, and analysis methods such as Density Functional Theory (DFT) or the method of Barrett, Joyner, and Halenda (BJH method), which is employed to extract pore size distributions from experimental isotherms based on the Kelvin model of pore filling.

For assessments of larger pores, mercury intrusion (pores sized from 3.2 nm to larger than 400 µm), and capillary flow porometry (pores sized from 13 nm to 500 µm) can be used.

Preferably, the homogeneous porous matrix of the present invention has:
- a maximum surface area from about 1 to about 1500 m²/g, and/or
- a pore size from 2 to 500 nm, preferably from 2 to 200 nm, more preferably ranging from 25 nm to 150 nm.

The "surface area" is advantageously obtained by applying the Brunauer-Emmett-Teller (BET) method to derive the surface area from physisorption isotherm data. According to ISO 9277-1995, the specific surface area is measured using a nitrogen measurement via the BET method.

The porosity can further be shown and visualized in Transmission Electron Microscopy (TEM) images. TEM images taken in bright field imaging mode show a variation in contrast between bright and dark areas within one displayed particle, the bright parts representing the regions where electrons interact less with the material. These regions correspond to pores in the material.

More preferentially, size of the pores is compatible with the size of information support.

In a preferred embodiment, the homogeneous porous matrix is composed of at least 90% w/w of salts, preferentially inorganic salts, consisting of:
- an ionic assembly of positively charged cations and negatively charged anions, or
- metal oxide, e.g., ZrO₂.

As use herein, mineral compounds (salts, metal oxides) are considered "insoluble" if less than 1 gram of it can be completely dissolved in 1L of targeted solvent. Tables of insoluble salts in water can be found in Techniques de l'Ingenieur "Solubilité dans l'eau des composes minéraux", Réf: K590 v1 (1992) https://doi.org/10.51257/a-v1-k590: based on J. H. Perry, Chemical Engineers Handbook*.* For insoluble compounds, solubility is given in milligrams of anhydrous substance per liter of solvent (generally water), as a function of temperature. For example, only 11.2 mg of CaCO3 is soluble in 1L of water at 18°C.

Anions are advantageously selected from carbonate, phosphate, sulfate, borate, silicate (e.g., CO₃²⁻, PO₄³⁻, SO₄²⁻, BO₃³⁻, SiO₃²⁻, etc.) or a combination thereof.

Cations are advantageously selected from calcium, magnesium, barium, aluminum, zinc, strontium, manganese (e.g., Ca²⁺, Mg²⁺, Ba²⁺, Al ³⁺, Zn²⁺, Sr2⁺, Mn²⁺ etc.) or a combination thereof.

Preferably, particles are mainly (preferably more than 90% w/w) composed of calcium carbonate, calcium phosphate, barium sulfate, barium phosphate, barium carbonate, borosilicate, calcium silicate, manganese carbonate or a combination thereof.

Calcium carbonate is preferably mesoporous calcium carbonate, i.e., the chemical element CaCO₃, X H₂O which has crystallized in the form of particles presenting a structural particularity organized in a network of channels of variable pore sizes.

Calcium carbonate porous particles are for example disclosed in:
- Volodkin DV, Petrov Al, Prevot M, Sukhorukov GB. Matrix polyelectrolyte microcapsules: new system for macromolecule encapsulation. Langmuir. 2004 Apr 13;20(8):3398-406. doi: 10.1021/la036177z. PMID: 15875874;
- Volodkin DV, Larionova NI, Sukhorukov GB. Protein encapsulation via porous CaCO3 microparticles templating. Biomacromolecules. 2004 Sep-Oct;5(5):1962-72. doi: 10.1021/bm049669e. PMID: 15360312;
- Anna Vikulina, Joseph Webster, Denis Voronin, Evgenii Ivanov, Rawil Fakhrullin, Vladimir Vinokurov, Dmitry Volodkin, Mesoporous additive-free vaterite CaCO3 crystals of untypical sizes: From submicron to Giant, Materials & Design, Volume 197, 2021, 109220, https://doi.org/10.1016/j.matdes.2020.109220;
- Ferreira AM, Vikulina AS, Volodkin D. CaCO3 crystals as versatile carriers for controlled delivery of antimicrobials. J Control Release. 2020 Dec 10;328:470-489. doi: 10.1016/j.jconrel.2020.08.061. Epub 2020 Sep 5. PMID: 32896611.

The homogeneous porous matrix is advantageously insoluble in the said material (inability of the matrix to form such a solution in said material).

The homogeneous porous matrix advantageously integrates other organic and/or inorganic chemical elements.

Said organic and/or inorganic chemical elements can advantageously influence particles properties, e.g., their sizes, their structures and their properties.

Said organic and/or inorganic chemical elements are for example chosen among short polymers, fatty acids, short organic molecules as ethylene glycol or other inorganic salts.

Particles are advantageously decomposed by conditions that are inert with respect to the information support stability, e.g.:
- metal chelating agents, e.g., EDTA (ethylenediaminetetraacetic acid), NTA (N,N-bis(carboxymethyl)glycine), or DTPA (diethylenetriaminepentaacetic acid), and/or
- specific pH conditions (as acidic or alkaline aqueous solutions).

As used herein, terms "decompose" or "decomposition", means advantageously that the homogeneous porous matrix is dissolved (in soluble salts or gas) using metal chelating agents, or the use of a specific pH conditions, to release the information support.

According to IUPAC (Muller, P. "Glossary of terms used in physical organic chemistry (IUPAC Recommendations 1994, Pure and Applied Chemistry, vol. 66, no. 5, 1994, pp. 1077-1184. https://doi.org/10.1351/pac199466051077), "chelation" relates to the formation or presence of two or more separate coordinate bonds between a polydentate (multiple bonded) ligand and a single central atom.

Usually these ligands are organic compounds, and are called chelants, chelators, chelating agents, or sequestering agents.

A metal chelating agent is a chemical compound that reacts with metal ions to form stable, water-soluble metal complexes. The agent rearranges the metal's chemical composition and improves the metal's general stability and likelihood to bond with other substances

For example, calcium carbonate porous particles can be dissolved with a 100 mM EDTA solution, or an aqueous solution with pH below 5, resulting from Ca²⁺ and CO₃²⁻ ions dissociation.

### Information support

The information support is added to said homogeneous porous matrix, wherein said information support consists of a polymer comprising an information sequence.

The information support may be loaded by adsorption, or co-precipitation, on and/or in the homogeneous porous matrix.

In other words, the information support is advantageously included within the pores, and/or on the surface, of the homogeneous porous matrix.

Also in other words, the information support is advantageously included, at least partially or entirely, within the pores of the homogeneous porous matrix.

The information sequence of said information support results advantageously from a transcoding of a system of ordered bits, the latter resulting from an encoding of a set of raw data.

As used herein, the "transcoding" means advantageously the use of codes to represent information in order to ensure the integrity of the information (detection and correction of errors) and to guarantee the security of the information (encryption/ciphering). This code therefore has preferably an error correction system.

An encryption method is for example disclosed in:
- Erlich et al., DNA Fountain enables a robust and efficient storage architecture, Science 355, 950-954 (2017),
- Cheng Kai Lim, Saurabh Nirantar, Wen Shan Yew, Chueh Loo Poh, Novel Modalities in DNA Data Storage, Trends in Biotechnology, Volume 39, Issue 10, 2021, Pages 990-1003. https://doi.org/10.1016/j.tibtech.2020.12.008, or
- Ceze, L., Nivala, J. & Strauss, K. Molecular digital data storage using DNA. Nat Rev Genet 20, 456-466 (2019). https://doi.org/10.1038/s41576-019-0125-3.

As used herein, the term "encryption method" means the process that converts the data into ordered binary codes and then transposes them into a chemical structure that will be the information support.

Nucleic acids such as DNA, including nucleic acids analogs, may be used to store digital information by designing a sequence of nucleotide bases that encodes the zeros and ones of the digital information. There are various techniques and encoding schemes known to those of skill in the art for using nucleotide bases to represent digital information.

Advantages of using nucleic acids and/or analogs, rather than another storage media for storing digital information, include information density and longevity.

In sequencing and synthesizing nucleic acids and/or analogs, substitution, insertion and deletion errors occur, so the data needs to be protected against these errors. In addition, environmental factors such as reactive oxygen species or UV radiation can alter nucleic acid sequences. Therefore, encoding and error correction codes such as Huffman codes or Reed-Solomon codes known to those of skill in the art can be used to counter these drawbacks.

According to encryption method, ordered binary-coded data are thus converted into a nucleic acid base sequence conversion resulting in the information sequence.

Finally, the encrypted data can be deciphered by determining the nucleic acid base sequence of the information sequence.

As used herein, the term "encrypted data" means advantageously each element of data (e.g., text, images, files) that can be encrypted or encoded in the information sequence and can be deciphered from information support recovered from the product and decoded or decrypted according to selected methods. The decoded or decrypted data can then be used to verify the properties of the material, or to authenticate the material, or to exclude counterfeit items.

Thus, in a preferred embodiment, the information support is chosen among nucleic acids and nucleic acids analogs, preferably DNA and more preferably single stranded DNA or double stranded DNA.

As used herein, the term "nucleic acid" means an oligomer composed of nucleotides, which can be deoxyribonucleotides or ribonucleotides. These compounds can be natural or synthetically produced deoxyribonucleotides or ribonucleotides. The synthetically produced nucleic acid can be of a naturally occurring sequence, or a non-natural sequence. The terms "ribonucleic acid" and "RNA" denote an oligomer composed of ribonucleotides. The terms "deoxyribonucleic acid" and "DNA" denote an oligomer composed of deoxyribonucleotides.

Terms "nucleic acid", "polynucleotide", "target polynucleotide", and "target nucleic acid" are used interchangeably.

As used herein, the term "nucleic acid analogs" means nucleic acids that may include one or more analogs (e.g., altered backbone, sugar, or nucleobases). Some non-limiting examples of analogs include: 5-bromouracil, peptide nucleic acids, xeno nucleic acids, morpholino compounds, locked nucleic acids, diol nucleic acids, threose nucleic acids, dideoxynucleotides, cordycepin, 7-deaza-GTP, fluorophores (e.g., rhodamine or fluorescein linked to a sugar), thiol-containing nucleotides, biotin-linked nucleotides, fluorescent base analogs, CpG islands, methyl-7-guanosine, methylated nucleotides, inosine, thiouridine, pseudouridine, dihydrouridine, stevioside, and tetanoside.

The information support consists preferably of nucleic acids fragments, preferably having a size of less than 500 bp, preferably ranging from 50 to 250 bp.

Advances in sequencing, the predominant form of information retrieval, or "reading", drive the viability of DNA data storage. Predominant methods of reading DNA include sequencing by synthesis (SBS), along with third-generation sequencing methods involving single-molecule sequencing via nanopores or enzymatic well-based reactions.

Preferably, the information sequence comprises an identifiable sequence, also named "detectable sequence", e.g. a nucleic acid or nucleic acids analogs sequence, which can be detected and sequence by a specific technology (e.g., hybridization, PCR technology, sequencing, capture, electrochemical detection).

### Fluidizing agent

As stated above, the particles have a particle surface having a coating with at least one fluidizing agent, suitable for promoting uniform dispersion (or homogeneous dispersion) of said particles within said material. In the present invention, "uniform dispersion" encloses also "homogeneous dispersion" or "random dispersion", advantageously without aggregates of particles.

"Uniform dispersion" advantageously means that the particles are evenly, or at least approximatively evenly, spaced or distributed within the material.

As used herein, dispersion is defined as a system in which distributed particles of one material are dispersed in a continuous phase of another material. The two phases may be in the same or different states of matter.

Dispersions are classified in several different ways, including how large the particles are in relation to the particles of the continuous phase, whether or not precipitation occurs, and the presence of Brownian motion.

In general, dispersions of particles sufficiently large for sedimentation are called suspensions, while those of smaller particles are called colloids and solutions. See "Terminology of polymers and polymerization processes in dispersed systems (IUPAC Recommendations 2011)". Pure and Applied Chemistry. 83 (12): 2229-2259, and Compendium of Polymer Terminology and Nomenclature (IUPAC Recommendations 2008) (2nd ed.). RSC Publ. p. 464 for IUPAC definition.

As used herein, the coating consists advantageously in a surface modification of said particles, i.e., an alteration of the physicochemical properties of a surface through the application of a chemical substance, preferably a surface coating, forming surface-treated particles.

Surface coating is advantageously a coverage of the surface of said particles with an adherent layer, i.e. said fluidizing agent.

As used herein, the fluidizing agent relates to a microparticle surface treating agent chosen to modify handling characteristics of the particles in the targeted material.

In the context of the invention, the term "fluidizing agent" relates advantageously to a functionalizing agent modifying the physico-chemical properties of the particles in order to improve their introduction into the target material.

The "fluidizing agent" is advantageously chosen among a crystallization inhibitor, a dispersant, a surface-treating agents, an agent for enhancing the fluidity of microparticles dispersions, or a combination thereof.

The at least one fluidizing agent is advantageously directly dependent on the nature of the targeted material, which can be notably a hydrophobic environment (oil, plastics, polymers) or an aqueous media (paint, thermosetting prepolymer).

In modifying surface properties of the particles, the fluidizing agent advantageously acts on at least one particles property and so can be measured, i.e.:
- change surface charge (zeta potential) modifying stability behavior and/or interactions with other components of the material,
- change in aggregation/sedimentation properties of the particles (alone in a solvent, or in interaction with other particles of the said materials),
- change in particles dispersibility if the targeted material is a liquid. As use herein, particles dispersibility corresponds to the ease to disperse a powder into a liquid, to reach spatial uniformity and the desired particle size (see ISO/TS 22107:2021),
- change in uniformity of particles dispersions if the targeted material is a solid (no particle aggregation).

The zeta potential of the particles has values that typically range from -100 to +100 mV. The magnitude of the zeta potential is predictive of the colloidal stability of the solution. The default zeta potential analysis limits are +150 to -150mV respectively. See Tech Note: Zeta potential quality report for the Zetasizer Nano.

For example, the zeta potential of porous calcium carbonate microparticles can be changed from negative to positive zeta potential by coating with a positive polymer.

Zeta potential of CaCO₃ porous microparticles is determined to be -12.2 mV in water at fixed pH. Adsorption of poly(allylamine hydrochloride) (PAH, Mw ~ 70 kDa) fluidizing agent onto the surface of particles is sufficient to reach a zeta potential value of + 10 mV (see Volodkin DV et al. Matrix polyelectrolyte microcapsules: new system for macromolecule encapsulation. Langmuir. 2004 Apr 13;20(8):3398-406. doi: 10.1021/la036177z. PMID: 15875874).

In another example, surface modification of nanosized precipitated calcium carbonate nanoparticles (nanoPCC) by fluidizing agents (e.g., alginate, pectin, acrylamide (A-PAM), or nanofibrillated cellulose (NFC)) resulted in a charge reversal indicated as a change in the sign of the zeta-potential from positive to negative.

Addition of NFC to the nanoPCC dispersion caused a charge reversal, changing the zeta-potential from approximately +10 mV to -20 mV at the fixed pH of the experiment.

The stability of the saturated dispersions was judged by measuring the change in turbidity, i.e., light transmitted through a sample with the Turbiscan Ma 2000 instrument.

Without modification the nanoPCC dispersion was unstable, seen as a rapid increase in light transmission due to sedimentation of flocculated particles (more than 40% in few minutes). The pectin modified nanoPCC and NFC modified nanoPCC dispersion was stable and no change in turbidity as a function of time was noted.

The stability of nanoPCC particles was also improved using A-PAM and alginate; no phase separation was detected, yet a slight increase (ΔTurbidity ~8%) in light transmission was observed.

Conversely, addition of 1 mL of 1% hydrophobising agent e.g., alkenyl succinic anhydride (ASA) to a dispersion of unmodified nanoPCC particles caused the mixture formed to aggregate and phase separate observed as a rapid increase in ΔTurbidity(%) reaching 80% in 120 min.

Such fluidizing agent ASA bearing a branched iso-alkenyl chain (C14 to C22), are widely used, especially in surface sizing of paper, paperboard, and cardboard, as well as in the hydrophobicization of cellulose fibers. This fluidizing agent destabilize particles in aqueous solvent but enhance particles dispersibility in hydrophobic environments such as organic solvents or plastics. (see Nypelö T et al. Tailoring Surface Properties of Paper Using Nanosized Precipitated Calcium Carbonate Particles. ACS Appl. Mater. Interfaces 2011, 3, 9, 3725-3731. https://doi.org/10.1021/am200913t).

The effects of fluidizing agents on particles can be measured by numerous techniques depending on the targeted parameter, well known in the prior art.

Regarding surface charge, the electrophoretic mobility of investigated samples can advantageously be measured by means of ZetaSizer Nano ZS (Malvern, UK) operating at the wavelength 633 nm. The equipment measures the electrophoretic mobility of the particles and converts it into the zeta potential (ZP), using the von Smoluchowski equation. The results are expressed as the average of at least three independent measurements.

Regarding hydrodynamic radius, the particle average hydrodynamic diameter, Dh, of particles is advantageously determined by DLS using a Zetasizer 3000 (Malvern, UK) equipped with a 10 mW He-Ne laser (633 nm) as light source. The measurements are performed at a scattering angle of 90°, and the reported results are the average of three independent measurements.

Regarding aggregation/sedimentation properties, an optical analyzer is advantageously used to study suspension behavior and to determine sedimentation speed (Turbiscan MA 2000, Formulaction, Toulouse, France). This instrument detects and measures modifications of concentrated and opaque suspensions. The Turbiscan carries out step-by-step vertical scanning of the whole sample by a pulsed near-infrared light source (λ= 850 nm) and converts the macroscopic aspects of the mixtures into two graphics (see Mengual O, Meunier G, Cayré I, Puech K, Snabre P. TURBISCAN MA 2000: multiple light scattering measurement for concentrated emulsion and suspension instability analysis. Talanta. 1999 Sep 13;50(2):445-56. doi: 10.1016/s0039-9140(99)00129-0. PMID: 18967735). Acquiring transmission and back scattering data come respectively from the transmission detector which receives the light going across the sample (at 0° from the incident beam), while the back scattering detector receives the light scattered backward by the sample at 135° from the incident beam. The stability of dispersions was judged by measuring the change in turbidity, i.e., light transmitted through a sample. Turbiscan gives the backscattered light flux (ΔTurbidity in %, relative to external standards) as a function of the sample height. Turbiscan data can be reported as a change in transmitted light versus time measured from the middle of the test tube. The turbidity measurement was started directly after dispersion preparation.

Regarding examine particles dispersibility in a liquid, Turbiscan is also used. The stability of the particles dispersions was assessed in terms of the Turbiscan stability index (TSI) values.

Regarding examine uniformity of particle dispersion in a solid material, particles can be directly observed using Scanning Electron Microscopy (SEM) or Atomic Force Microscopy (AFM).

Regarding recrystallisation properties, stabilization of instable porous vehicles can advantageously be directly observed by microscopy techniques such as optical microscopy or scanning electron microscopy depending on their size.

Regarding surface area of microparticles, it is advantageously standard practice to apply the Brunauer-Emmett-Teller (BET) method to derive the surface area from physisorption isotherm data. According to ISO 9277-1995, the specific surface area was measured using a nitrogen measurement via the BET method.

Regarding surface energy, the contact angle is advantageously measured by a video optical contact angle tester (CA; OCA25, Eastern-Dataphy, Germany).

In practice, fluidizing agents include advantageously a large variety of molecules including the coating with organic molecules such as fatty acids, polymers, resins and/or coating with inorganic salts.

In a preferred embodiment, said at least one fluidizing agent is chosen among surface-treating agents of inorganic fillers, including:
- saturated fatty acids, advantageously having a carbon chain length of from C15 to C20, e.g., palmitic acid and stearic acid,
- unsaturated fatty acids, advantageously having a carbon chain length of from C15 to C20, e.g., oleic acid and linoleic acid,
- phospholipids, e.g., phosphatidylcholine, phosphatidylethanolamine, phosphatidylserine,
- lipids mixtures, preferably biosourced or biodegradable, e.g., lecithin, beeswax,
- resin acids, e.g., alicyclic carboxylic acids and abietic acid as well as salts, esters and ethers thereof,
- polymers, e.g., polycarboxylic acids, fused phosphoric acid, polyacrylate, polyacrylamide, polyamine, poly-aminoacyls, polystyrene, polysaccharide (dextran, alginate, chitosan, chondroitin, cellulose, pectin, etc.), lignin, shellac, preferably biosourced or biodegradable polymers (see Vroman I et al. Biodegradable Polymers. Materials (Basel). 2009;2(2):307-344. Published 2009 Apr 1. doi:10.3390/ma2020307),
- others inorganic salts,
- titanates, silanes, siloxanes or alkenyl succinic anhydrides surface treating agents.

As used herein, according to IUPAC Recommendations 2003, "biodegradable polymers" are polymers susceptible to degradation by biological activity, with the degradation accompanied by a lowering of its molar mass.

For example, the particles may be treated or coated with a hydrophobising agent such as, e.g., aliphatic carboxylic acids, salts or esters thereof, or a siloxane.

Suitable aliphatic acids are, for example, C5 to C28 fatty acids such as stearic acid, palmitic acid, myristic acid, lauric acid, or a mixture thereof.

The particle may also be treated or coated to become cationic or anionic with, for example, a polyacrylate or polydiallyldimethylammonium chloride (polyDADMAC).

### Synthesis of particles of the marking composition

As shown in the Examples, information support and fluidizing agent are advantageously incorporated simultaneously from solution onto the homogeneous porous matrix.

For example, information support and fluidizing agent are advantageously incorporated simultaneously by direct adsorption of macromolecules, from solution onto preformed homogeneous porous matrix (physical adsorption).

In an alternative embodiment, depending on the nature of the "fluidizing agent", information support and fluidizing agent can also be sequentially added to the porous vehicle.

### Material

The present invention also relates to the material comprising the marking composition according to the invention.

In general, the material is advantageously chosen among liquid, semi-solid or solid materials, for example plastic, thermoset, varnish, rubber, paint, oil, pharmaceuticals.

Said material can be any solid traceable item, such as an electronic device, an item of clothing, paper, fiber, or fabric, or any other item of commerce, or cash or valuables, whether in storage or in transit.

In addition, the item of commerce to be marked with said molecular tags can be a liquid, such as an ink, an oil, a dye or a spray.

As an item of commerce, the product can be a commodity item, such as paper, metal, wood, a plastic, rubber or a powder.

Moreover, the material can be pharmaceutical products or food products.

For example, the material is a viscous aqueous prepolymer which can be irreversibly cured by heating leading to the formation of a thermoset ("a cured thermosetting polymer", see PAC, 2004, 76, 889. (Definitions of terms relating to reactions of polymers and to functional polymeric materials (IUPAC Recommendations 2003)) on page 898.).

In contrast, the targeted material may be a thermoplastic material. Thermoplastic materials are transformed without chemical reaction as opposed to thermosetting polymers. No thermal degradation appears, and the molecular structure of the polymer is not modified during transformation. Thermoplastics are advantageously defined in "ISO 7792-1:2012, Plastics - Thermoplastic polyester (TP) molding and extrusion materials - Part 1: Designation system and basis for specifications".

In a preferred embodiment, the step of incorporating consists advantageously of incorporating the marking composition into the material during the fabrication process of said material.

In other words, the present invention also relates to a fabrication process of a material comprising a step of incorporating said marking composition into said material during its fabrication process.

Preferably, the material is batches generated. As used herein, "batch generated" relates advantageously to a method of manufacturing where the material is made as specified groups or amounts, within a period.

A batch goes advantageously through a series of steps in a large manufacturing process to make the final desired material.

In general, the concentration of marking composition is advantageously from 0.5 to 150 mg of marking composition per L or kg of material.

In general, the particles of said marking composition are advantageously intact in said material of interest.

### Extraction and reading of the information support

The material is advantageously treated to extract the particles and then to extract the information support from said extracted particles.

The information sequence is advantageously retrieved from the material through a "information sequence extraction method", i.e., the processes by which the information sequence is obtained from the material.

The particles can thus be treated by decomposition condition as disclosed before (metal chelating agents or specific pH conditions), to specifically release the informational support.

For example, calcium carbonate homogeneous porous matrix can be treated with an acidic solution (pH below 5) or an EDTA solution to release the informational support.

Thus, in general, the extraction method is advantageously performed by dissolving the homogeneous porous matrix, e.g., in an aqueous solution of complexing agent, followed by extraction and purification steps to recover the information support.

Finally, a reading step corresponds to the sequencing of an information support and the decoding of the information sequence via the selected encoding method.

Various other modifications may be made to the invention within the scope of the appended claims.

### Examples

### Synthesis of the mesoporous vaterite microparticles (vehicle).

In a typical experiment, 0.33 M Na₂CO₃ solution (50 mL) was rapidly poured into an equal volume of 0.33 M solution of CaCl₂ (50 mL) at 20°C, and after 30 seconds intense controlled agitation (650 rpm) with a digital Electronic Overhead Stirrer the white precipitate was filtered off on Satorlon Polyamid filter (pore size 0.2 µm).

Thoroughly, the precipitate was washed two times with pure water to remove excess of ions, and then two times with 100% ethanol to improve dispersion characteristics.

The obtained powder was dried at 55°C for about 12 hours. The procedure results in highly homogeneous, spherical CaCO₃ microparticles with an average diameter ranging of 6 µm.

The size of microparticles can be modified by changing some parameters as salts concentrations, agitation speed or temperature.

In another typical experiment, 0.6 M Na₂CO₃ solution (50 mL) was rapidly poured into an equal volume of 0.6 M solution of CaCl₂ (50 mL) at 20°C. After 30 seconds intense controlled agitation (650 rpm) with a digital Electronic Overhead Stirrer, the white precipitate was filtered off on Satorlon Polyamid filter (pore size 0.2 µm).

Applying the same filtration and drying procedure, results in highly homogeneous, spherical CaCO₃ microparticles with an average diameter of 1.5 µm.

### Generation and synthesis of informational support DNA sequences.

Short barecode coding DNA sequences (typically 10 bp to 210 bp) were computer generated to introduce the digital information composed of alphanumerical characters.

For example, the digital information can be encoded in nucleobases (barecode coding sequences) via a Reed-Solomon error-correcting code algorithm. This format allows the storage of digital information in polynucleotide molecules.

On both sides of this barecode coding sequence, 2x20 bp orthogonal (non-interacting) polymerase chain reaction primers binding regions are generated for amplifying the DNA sequence. The association of barecode coding sequence plus 2 x 20 bp primers binding regions on both sides give the informational support sequence.

In a specific embodiment aimed at stabilizing the DNA molecule, the DNA strand complementary to the informational support sequence carrying the digital information can be generated.

Finally, double strand DNA informational supports (ranging to 50 bp to 250 bp) are chemically synthesized.

### Loading of informational support and fluidizing agent (surface functionalization agent):

Double strand nucleotidic informational support and fluidizing agent are incorporated by direct adsorption of macromolecules from solution onto preformed CaCO3 microspheres (physical adsorption).

In a typical experiment, DNA at 10 µM concentration and the sodium polyacrylate salt (fluidizing agent) at 0.5% (w/w) relative to mass of CaCO₃ microparticles are dissolved in loading buffer (KCI 0.5 N, pH 6.5).

Then 50 mg of CaCO₃ microparticles are resuspended in 2 ml Eppendorf tube containing 500 µL of loading buffer, vigorously vortexed and incubated under agitation for at least 4 hours.

It is important to note that pH of the loading buffer is chosen to favor interaction of particles with negatively charged macromolecules because the zeta-potential of microparticles was changed at pH between 8.0 and 9.0 from positive to negative values that can be explained by an excess of Ca²⁺. So, at pH 6.5, microparticles have a global net charge with positive value.

After incubation, the microparticles were centrifuged to remove the loading buffer and washed 1 time (for 5 min) with clean KCI 0.5 N pH 6.5 buffer to remove non adsorbed macromolecules. The microparticles were washed two times with 70 % ethanol solution and dried at 55°C in hood.

It possible to determine indirect DNA loading by measuring the disappearance of DNA absorbance (at 260 nm) in loading buffer and the washing buffer.

In a typical experiment, 6 µm molecular tags are loaded at concentration ranging from 0.5 to 1 µg of DNA per mg of inorganic vehicle.

In another typical experiment, 1.5 µm molecular tags are loaded at concentration ranging from 2.4 to 15 µg of DNA per mg of inorganic vehicle depending on the DNA loading solution concentration.

### Direct liberation of nucleotidic informational support from microparticles and amplification:

One of the particular advantages of calcium carbonate microparticles is that they can be easily decomposed and removed with an acidic pH (< 5) or by the addition of chelating agents such as EDTA.

Therefore, in typical experiment, microparticles are decomposed with a "decomposition buffer" (EDTA 100 mM, pH 5).

The decomposition of particles is almost instantaneous as evidenced by the appearance of CO₂ bubbles from the dissociation of particles.

After 5 min of vigorous agitation, informational nucleotidic support can be easily purified via commercial DNA purification kit (NEB PCR Clean up up or Macherey-Nagel NucleoSpin Gel and PCR Clean-up) and then amplified by PCR.

At the end, to extract the digital information, one only has to sequence the DNA fragments that were amplified with a polynucleotide sequencer.

### Incorporation of molecular tags in a thermoset polymer:

The base formulation for the thermoset was kindly provided by an industrial partner. Due to property rights, the detailed composition cannot be disclosed. In typical experiments, preloaded molecular tags are directly diluted in an alkaline aqueous thermosetting prepolymer with a selected dilution factor (for example 50 mg of molecular tags per L of prepolymer).Then, the aqueous mixture is vigorously agitated to favor microparticles dispersion and homogenization.

Later, the curing of the thermosetting prepolymer (polymerization and crosslinking of the prepolymer) containing molecular tags can be achieved by heating treatment (3 hours at 100°C) leading to the formation a 'tagged" thermoset.

The resulting polymerized insoluble thermoset is a solid. Typically, 250 µl of heated thermosetting prepolymer give 50 mg of solid thermoset.

### Extraction and amplification from the cured thermoset:

The polymerized thermoset can be treated to extract informational nucleotidic support.

Briefly, 50 mg of the solid is shredded in micrometric particles with a cryogenic grinder (CryoMill, RETSH) to favor contact between "decomposition buffer" (EDTA 100 mM, pH 5) and molecular tags.

For the same reason, solid thermoset micrometric particles (50 mg) are "pre-swelled" in 600 µL of chloroform (or toluene) for 1 hour.

Then 200 µL of "decomposition buffer" is added and the sample is vigorously agitated at least one hour to decompose calcium carbonate vehicle. To enhance DNA purification yield for much diluted DNA samples, polyA from Sigma is added in "decomposition buffer" at 1 µg/mL during the extraction. Poly(A) is used as a carrier for quantitative precipitation or purification of DNA and RNA.

After decantation, in the case of chloroform, the nucleotidic informational support is in the upper aqueous phase while hydrophobic constituents that can perturb PCR reactions are in the lower phase.

To eliminate EDTA that will also inhibit PCR reactions, the aqueous phase containing the informational support is purified by classical DNA purification kit (NEB PCR Clean up) prior to PCR reactions that permit to amplify the information.

Purified DNA can be then sequenced.

### Incorporation of molecular tags in a biodegradable thermoplastic (such as Polylactic Acid (PLA) or PolyCaproLactone (PCL) or DL-lactide and glycolide copolymer (PDLG) or L-lactide and ε-caprolactone copolymer (PLC)):

GMP grade thermoplastics polymers and copolymers are purchased from Corbion Purac. In typical experiments, PDLG 7507 (DL-lactide and glycolide copolymer in a 75:25 molar ratio) granules were completely dissolved in chloroform at a concentration of 10 wt % (10 g of copolymer diluted in 100 mL of chloroform) by stirring for 60 min at 40°C. 1 mg of preloaded molecular tags (10 µg of informational DNA per mg of CaCO₃ microbeads) are diluted in 1 mL of ethanol, vortexed and then directly incorporated in the 10 % w/v copolymer/chloroform solution under vigorous mixing (10 min). Having done this stage, the prepared mixture was cast into clean Teflon Petri dishes, and chloroform was allowed to evaporate at ambient temperature and pressure under chemical hood. The obtained solid films were finally dried at 60°C in an oven for 48 h. The final concentration of informative DNA (10 µg in 10g of copolymer) corresponds to 1ppm.

### Extraction and amplification of the informational support from the thermoplastic:

Solid thermoplastics (dried polymers or copolymers) can be treated to extract informational nucleotidic support. Briefly, 30 mg of solid was cut and put into a vial. Then, 500 µL of chloroform was added into the vial, and polymer was re-solubilized under vigorous agitation. Subsequently, 500 µL of "decomposition buffer" (EDTA 100 mM, pH 5, complemented with 1 µg of polyA carrier) was added and the vial was stirred for 1 hour. After decantation, the nucleotidic informational support is in the upper aqueous phase, so 300 µL of the aqueous phase were taken using a sterile syringe and DNA was subsequently purified using Macherey-Nagel NucleoSpin Gel and PCR Clean-up kit.

Purified DNA can be then sequenced.

### Incorporation of molecular tags in a commercially available nail polish and informational support extraction:

In typical experiments, preloaded 1.5 µm molecular tags are diluted among same unloaded vehicle microparticles with a selected dilution factor (1% w/w for example). Then this mixture is directly incorporated in a 5 mL commercially available nail polish ("Go Green"^{®} by Yves Rocher) at a selected dilution (5 mg for 5 mL of nail polish for example) and vigorously agitated to favor molecular tags dispersion. Subsequently, nail polish is applied on ceramics spheres (MP Biomedicals^{™} 1/4 in. Ceramic Sphere) and let dry under a hoven overnight. Concretely, after drying (evaporation of nail polish solvents as ethyl acetate), the dry mass represents 30% of varnish deposited mass.

Next, for extraction, colored ceramic spheres are directly put in ethyl acetate solvent (400 µL) and agitated leading to the discoloration of ceramic spheres. Later, 200 µL of "decomposition buffer" (100 mM EDTA, pH 5 supplemented with 1 µg of Poly(A)) is added and the sample is vigorously agitated at least one hour to decompose calcium carbonate vehicle.

After decantation, the nucleotidic informational support is in the lower aqueous phase while hydrophobic constituents that can perturb PCR reactions are in the lower phase. Lastly, the informational nucleotidic support is purified from aqueous lower phase by classical DNA purification kit (NEB PCR Clean up) prior to PCR reactions that permit to amplify the information.

### DNA sequencing and digital data reading:

Amplified and purified polynucleotidic informational support can be sequenced with a polynucleotide sequencer.

Finally, alphanumerical data can be read and re-extracted from the nucleotidic sequence by decrypting the digital information with the computational algorithm selected in example 2.

## Claims

1. Method for marking a material, for example for purposes of authentication and/or traceability of said material, notably batches generated,
wherein said marking method comprises:
- a step of supplying a marking composition chosen among molecular tags, and
- a step of incorporating said marking composition into said material,
**characterized in that** said marking composition comprises particles, advantageously remaining intact after incorporation in said material, which comprise:
- a homogeneous porous matrix which comprises a plurality of pores,
- an information support added to said homogeneous porous matrix, wherein said information support consists of a polymer comprising an information sequence, and
- a particle surface having a coating with at least one fluidizing agent, suitable for promoting uniform dispersion of said particles within said material.

2. Marking method according to claim 1, **characterized in that** the homogeneous porous matrix is composed of at least 90% w/w of salts, preferentially inorganic salts, consisting of an ionic assembly of positively charged cations and negatively charged anions, or consisting of metal oxide, wherein said homogeneous porous matrix is insoluble in the said material.

3. Marking method according to any one of the claims 1 or 2, **characterized in that** the particles are decomposed by metal chelating agent or by specific pH conditions.

4. Marking method according to any one of the claims 1 to 3, **characterized in that** the particles have a size ranging from 200 nm to 100 µm, preferably from 500 nm to 10 µm, more preferably from 750 nm to 3 µm.

5. Marking method according to any one the claims 1 to 4, **characterized in that** the particles have pore size ranging from 2 nm to 500 nm, preferably from 2 to 200 nm, more preferably ranging from 25 nm to 150 nm.

6. Marking method according to any one of the claims 1 to 5, **characterized in that** the information support is included within the pores and/or absorbed on the surface of the homogeneous porous matrix.

7. Marking method according to any one of the claims 1 to 6, **characterized in that** the information sequence of said information support results from a transcoding of a system of ordered bits, the latter resulting from an encoding of a set of raw data.

8. Marking process according to any one of the claims 1 to 7, **characterized in that** the information support is chosen among nucleic acids and nucleic acids analogs, preferably DNA and more preferably single stranded DNA or double stranded DNA.

9. Marking method according to claim 8, **characterized in that** the information support consists of nucleic acids fragments, preferably having a size of less than 500 bp, preferably ranging from 50 to 250 bp.

10. Marking method according to any one of the claims 1 to 9, **characterized in that** the information support is associated, preferably adsorbed and/or included, with the said homogeneous porous matrix.

11. Marking method according to any one of the claims 1 to 10, **characterized in that** fluidizing agent is chosen among surface-treating agents of inorganic fillers, including saturated fatty acids; unsaturated fatty acids; phospholipids; lipids mixtures; resin acids; polymers; others inorganic salts, titanates, silanes, siloxanes, or alkenyl succinic anhydride surface treating agents.

12. Marking method according to any one of the claims 1 to 11, **characterized in that** the material is chosen among liquid, semi-solid or solid materials, for example plastic, thermoset, varnish, rubber, paint, oil, pharmaceuticals.

13. Marking method according to any one of the claims 1 to 12, **characterized in that** the step of incorporating consists of incorporating the marking composition into the material during the fabrication process of said material.

14. Marking composition, for marking a material, for example for purposes of authentication and/or traceability of said material, notably batches generated,
wherein said marking composition comprises particles, advantageously remaining intact after incorporation in said material, which comprise:
- a homogeneous porous matrix which comprises a plurality of pores,
- an information support added to said homogeneous porous matrix, wherein said information support consists of a polymer comprising an information sequence, and
- a particle surface having a coating with at least one fluidizing agent, suitable for promoting uniform dispersion of said particles within said material.

15. Material comprising a marking composition according to claim 14 or a marking composition obtained by a marking method according to any one of the claims 1 to 13.
